# EUROPÄISCHE PATENTSCHRIFT

(11) **EP 0 285 906 B1**
(45) Veröffentlichungstag und Bekanntmachung des Hinweises auf die Patenterteilung: **11.08.1993**
(21) Anmeldenummer: 88104603.1
(22) Anmeldetag: 23.03.1988
(51) Int. Cl.: A61K 31/275

(54) **Wirkstoff zur Tumorbekämpfung**
Active agent for combating tumours
Agent actif pour combattre les tumeurs

(30) Priorität: 31.03.1987 DE 3710806
(43) Veröffentlichungstag der Anmeldung: 12.10.1988
(73) Patentinhaber: BASF Aktiengesellschaft, 67063 Ludwigshafen (DE)
(72) Erfinder: Merry, Stephen Dr., Blanefield Glasgow 963 9 JF (GB)

(56) Entgegenhaltungen:
- EP-A- 0 075 823
- EP-A- 0 268 100
- DIALOG 0160440, Dehaen Drug Data 1980-1988, Subfile: Dir, Pharma 1982; "Norverapamil"

## Beschreibung

Aus der DE-B 1 158 043 (vgl. Beispiel 3b) ist das α-Isopropyl-α-(N-homoveratryl-γ-aminopropyl)-3,4-dimethoxyphenylacetonil (nachfolgend als Norverapamil bezeichnet) bekannt. Es ist dort als Zwischenprodukt zur Herstellung von Verapamil beschrieben.

In der JP-A 83 624/1983 ist die Verwendung von Verapamil zur Wirkverstärkung von Antitumormitteln beschrieben. A.B. Benson u.a. (Cancer Treat. Rep. 69, 795 (1985) fanden jedoch, daß die Kombination von Verapamil und Antitumormitteln nur beschränkt eingesetzt werden kann, da die kardiale Eigenwirkung von Verapamil eine erforderliche höhere Dosierung ausschließt. Analoges gilt für die Behandlung von Tumormetastasen (EP-A 159 678) mit Verapamil alleine.

Überraschenderweise wurde nun gefunden, daß sich das Norverapamil gut zur Tumorbehandlung Zusammen mit einem Cancerostaticum verwenden läßt, da seine kardiovaskuläre Wirkung deutlich schwächer ausgeprägt ist (Cardiovasc. Res. 12, 247 (1978)) und es deshalb in ausreichend hohen Dosen appliziert werden kann.

Norverapamil kann - falls gewünscht - in Form seiner Salze mit physiologisch verträglichen Säuren eingesetzt werden. Als physiologisch verträgliche Säuren kommen vorzugsweise in Betracht: Salzsäure, Schwefelsäure, Phosphorsäure, Essigsäure, Zitronensäure, Malonsäure, Salicylsäure, Maleinsäure, Fumarsäure, Bernsteinsäure, Ascorbinsäure, Äpfelsäure, Methansulfonsäure, Milchsäure, Gluconsäure, Glucuronsäure, Amidosulfonsäure, Benzoesäure, Weinsäure.

Die Dosierung des Norverapamils als Antimetastasen-Mittel ist je nach Art des Krebses und des Krankheitszustands verschieden. In der Regel beträgt die tägliche Dosis beim Erwachsenen pro Tag 300 bis 1000 mg bei oraler Gabe, bei intravenöser Gabe 200 bis 300 mg und bei intraperitonealer Gabe 200 bis 500 mg.

Norverapamil kann in Form von Tabletten, Kapseln oder Dragees zur oralen Applikation oder als Injektionslösung zur parenteralen (i.v., i.p. i.m.) Applikation vorliegen. Lösungen können auch infundiert werden. Die Herstellung der Applikationsformen geschieht in bekannter Weise nach üblichen Methoden.

Norverapamil kann auch Patienten verabfolgt werden, die bereits anderen Tumortherapien - z.B. Chemotherapie, Endokrintherapie, Immuntherapie, Bestrahlung oder Chirugie - unterworfen worden sind oder unterworfen werden, und bei denen aufgrund von Resistenzerscheinungen die Wirkung von bekannten Tumormitteln, die wegen Nebenwirkungen nicht höher dosiert werden können, nachgelassen hat.

Norverapamil kann auch zur Wirkungsverstärkung von Antitumormitteln (Cancerostatica) verwendet werden.

Die Erfindung betrifft Erzeugnisse enthaltend Norverapamil und ein Cancerostaticum als Kombinationspräparat zur gleichzeitigen, getrennten oder zeitlich abgestuften Anwendung in der cyclostatischen Therapie.

Als Cancerostatica kommen insbesondere in Betracht:
a) Antibiotica wie Actinomycin D, Doxorubicin (Adriamycin), Daunorubicin, Mithramycin und Bleomycin sowie andere interchalatorisch wirkende Substanzen, wie Amonafide und Mitonafide,
b) Alkaloide wie Vincristin, Vinblastin, Vindesin, Etoposid und Teniposid,
c) alkylierend wirkende Substanzen, wie Cyclophosphamid, Nitrosoharnstoffe und Cisplatin und
d) Antimetabolite wie Methotrexat, 5-Fluoruracil und dessen Analoge, 6-Mercaptopurin, 6-Thioguanin und Cytarabin.

Die oben genannten Substanzen können gewünschtenfalls in Form ihrer Salze mit physiologischen Säuren bzw. Basen vorliegen. Als physiologisch verträgliche Säuren kommen vorzugsweise die bereits genannten in Betracht.

Als physiologisch verträgliche Basen eignen sich insbesondere Ammoniak, Alkalimetallhydroxide - insbesondere des Natriums, Kaliums und Lithiums-, Erdalkalimetallhydroxide - besonders des Calciums und Magnesiums-, sowie organische Basen wie niedere Alkylamine - z.B. Methylamin oder Ethylamin-, Cyclohexylamin, substituierte niedere Alkylamine - z.B. Diethanolamin, Triethanolamin, Tris-(hyroxymethyl)-aminomethan -, Piperidin oder Morpholin.

Durch die neuen Kombinationen wird nicht nur das Wachstum von Krebszellen, sondern auch die Bildung von Metastasen verhindert oder stark eingeschränkt.

Norverapamil kann zusammen oder getrennt mit den Cancerostatica verabfolgt werden. Bevorzugt ist jedoch die getrennte, vorherige Applikation. Norverapamil wird in der Regel oral appliziert, während die Cancerostatica oral oder parenteral (z.B. i.v., i.p.) gegeben werden.

Das Verhältnis von Norverapamil zu Cancerostaticum hängt von der zu behandelnden Krebsart, dem Krankheitszustand des Patienten und dem verwendeten Cancerostaticum ab. In der Regel beträgt das Verhältnis 1:1 bis 500:1. Dabei werden das Norverapamil in der Regel in einer Menge von 200 bis 1000 mg pro Patient und Tag bei oraler Gabe und 200 bis 300 mg bei intravenöser bzw. 200 bis 500 mg bei intraperitoneale Gabe pro Patient und Tag angewendet. Die Cancerostatica werden in der Menge appliziert, die auch für die alleinige Gabe dieser Substanzen vorgesehen ist und die z.B. aus der "Rote Liste 1986" bzw. den darin erwähnten wissenschaftichen Prospekten entnommen werden kann.

Die Substanzen können in Form von Tabletten, Kapseln oder Dragees zur oralen Applikation oder als Injektionslösung zur parenteralen (i.v., i.p., i.m.) Applikation vorliegen. Lösungen können auch infundiert werden. Die Herstellung der Applikationsformen geschieht in bekannter Weise nach üblichen Methoden.

### Beispiele

1. Es werden in üblicher Weise Oblongtabletten folgender Zusammensetzung hergestellt:
   500 mg Norverapamil
   120 mg Lactose
   60 mg Cellulose
   3 mg Magnesiumstearat
   50 mg Maisstärke
   15 mg Polyvinylpyrrolidon.
2. 3,0 mg Vincristinsulfat und 500 mg Norverapamil werden in 250 ml physiologischer Kochsalzlösung gelöst, sterilisiert und unter sterilen Bedingungen in eine Infusionsflasche gefüllt.
3. 10 Ampullen mit je 1,5 mg Mithramycin "Pfizer" (vgl. Rote Liste 1985, Nr. 85 038) und eine Durchdrückpackung mit 10 Oblongtabletten mit Norverapamil-Hydrochlorid wurden zusammen in eine Schachtel gepackt. Die Oblongtabletten waren in üblicher Weise hergestellt worden und enthielten pro Tablette 500 mg Norverapamil, 120 mg Lactose, 60 mg Cellulose, 3 mg Magnesiumstearat, 50 mg Maisstärke und 15 mg Polyvinylpyrrolidon.

## Patentansprüche (Patentansprüche für folgende(n) Vertragsstaat(en): AT, BE, CH, DE, FR, GB, IT, LI, NL)

1. Erzeugnisse enthaltend Norverapamil und ein Cancerostaticum als Kombinationspräparat zur gleichzeitigen, getrennten oder zeitlich abgestuften Anwendung in der cytostatischen Therapie.

2. Erzeugnisse gemäß Anspruch 1, dadurch gekennzeichnet, daß sie Norverapamil und ein Cancerostaticum im Verhältnis von 1:1 bis 500:1 enthalten.

## Patentansprüche (Patentansprüche für folgende(n) Vertragsstaat(en): ES)

1. Erzeugnisse enthaltend Norverapamil und ein Cancerostaticum als Kombinationspräparat zur gleichzeitigen, getrennten oder zeitlich abgestuften Anwendung in der cytostatischen Therapie.

2. Erzeugnisse gemäß Anspruch 1, dadurch gekennzeichnet, daß sie Norverapamil und ein Cancerostaticum im Verhältnis von 1:1 bis 500:1 enthalten.

3. Verfahren zur Herstellung eines Antitumormittels, dadurch gekennzeichnet, daß man Norverapamil und ein Cancerostaticum zusammenbringt.

## Claims (Claims for the following Contracting State(s): AT, BE, CH, DE, FR, GB, IT, LI, NL)

1. A product containing norverapamil and a cancerostatic as a combination preparation for simultaneous, separate or sequential use in cytostatic therapy.

2. A product as claimed in claim 1, which contains norverapamil and a cancerostatic in a ratio of 1:1 to 500:1.

## Claims (Claims for the following Contracting State(s): ES)

1. A product containing norverapamil and a cancerostatic as a combination preparation for simultaneous, separate or sequential use in cytostatic therapy.

2. A product as claimed in claim 1, which contains norverapamil and a cancerostatic in a ratio of 1:1 to 500:1.

3. A process for the preparation of an antitumor agent, wherein norverapamil and a cancerostatic are combined.

## Revendications (Revendications pour l'(les) Etat(s) contractant(s) suivant(s): AT, BE, CH, DE, FR, GB, IT, LI, NL)

1. Produits contenant du norvérapamil et un cancérostatique à titre de préparation de combinaison pour l'utilisation simultanée, séparée ou échelonnée dans le temps en vue d'une thérapie cytostatique.

2. Produits suivant la revendication 1, caractérisés en ce qu'ils contiennent le norvérapamil et un cancérostatique en une proportion de 1:1 à 500:1.

## Revendications (Revendications pour l'(les) Etat(s) contractant(s) suivant(s): ES)

1. Produits contenant du norvérapamil et un cancérostatique à titre de préparation de combinaison pour l'utilisation simultanée, séparée ou échelonnée dans le temps en vue d'une thérapie cytostatique.

2. Produits suivant la revendication 1, caractérisés en ce qu'ils contiennent le norvérapamil et un cancérostatique en une proportion de 1:1 à 500:1.

3. Procédé de préparation d'un agent antitumoral ou d'une composition antitumorale, caractérisé en ce que l'on rassemble du norvérapamil et un cancérostatique.
